# EUROPÄISCHE PATENTSCHRIFT

(11) **EP 0 576 451 B1**
(45) Veröffentlichungstag und Bekanntmachung des Hinweises auf die Patenterteilung: **29.05.1996**
(21) Anmeldenummer: 92905644.8
(22) Anmeldetag: 07.03.1992
(51) Int. Cl.: C07C 69/704, C07H 15/04, C07C 219/06

(54) **CITRONENSÄURE-ESTER VON POLYHYDROXYVERBINDUNGEN UND IHRE VERWENDUNG IN WASCH- UND REINIGUNGSMITTELN**
CITRIC ACID ESTERS OF POLYHYDROXYLE COMPOUNDS AND THEIR USE IN WASHING AND CLEANING PRODUCTS
ESTERS D'ACIDE CITRIQUE DE COMPOSES POLYHYDROXYLES ET LEUR UTILISATION DANS DES PRODUITS DE LAVAGE ET DE NETTOYAGE

(30) Priorität: 16.03.1991 DE 4108626
(43) Veröffentlichungstag der Anmeldung: 05.01.1994
(73) Patentinhaber: BASF Aktiengesellschaft, D-67063 Ludwigshafen (DE)
(72) Erfinder: KAPPES, Elisabeth, D-6800 Mannheim 1 (DE); OFTRING, Alfred, D-6702 Bad Duerkheim (DE); BAUR, Richard, D-6704 Mutterstadt (DE); KUD, Alexander, D-6509 Eppelsheim (DE); BOECKH, Dieter, D-6703 Limburgerhof (DE); HARTMANN, Heinrich, D-6703 Limburgerhof (DE); SCHWENDEMANN, Volker, D-6730 Neustadt (DE)
(86) Internationale Anmeldenummer: EP9200512
(87) Internationale Veröffentlichungsnummer: WO9216493

(56) Entgegenhaltungen:
- EP-A- 0 258 814
- WO-A-91/16296
- GB-A- 894 752
- JP-A-52 049 203
- US-A- 3 661 955

## Beschreibung

Die Erfindung betrifft Ester aus Citronensäure oder Acetyl-CitronensäureAnhydrid und mindestens 3 OH-Gruppen enthaltenden Verbindungen aus der Gruppe Polyglycerin, Zuckercarbonsäuren, Alkylglucoside, Mono-, Oligo- und Polysaccharide, Chitin, Chitosan, Aminosorbit, Glucosamin, Triethanolamin und Trishydroxyethylmelamin und ihre Verwendung als Zusatz zu phosphatarmen oder phosphatfreien Wasch- und Reinigungsmitteln.

Aus der DE-B-2 147 778 sind Wasch- und Reinigungsmittel bekannt, die wasserlösliche Salze von sauren Carbonsäureestern aus mindestens dreiwertigen aliphatischen oder olefinisch ungesättigten Carbonsäuren bzw. Hydroxycarbonsäuren und mindestens dreiwertigen aliphatischen Alkoholen als Gerüststoffe aufweisen, wobei an jede Hydroxylgruppe des Alkohols ein Molekül der Carbonsäure gebunden ist. Typische Beispiele für solche Ester sind Glycerintricitrat, Sorbithexacitrat und Pentaerythrittetracitrat. Diese Citronensäure-Ester sind biologisch gut abbaubar, wirken in Wasch-und Reinigungsmitteln als Gerüststoffe und bilden in der Waschflotte mit hydrophilen Pigmentteilchen stabile Dispersionen.

Aus der DE-B-1 617 122 sind Wasch- und Reinigungsmittel bekannt, die durch einen Gehalt von 0,1 bis 20 Gew.-%, bezogen auf das Gesamtgewicht der Mittel, an wasserlöslichen Salzen von freie Carboxylgruppen enthaltenden Polyestern gekennzeichnet sind, deren Säurereste von Tricarbonsäuren und/oder Tetracarbonsäuren und deren Alkoholreste sich von zweiwertigen Alkoholen ableiten. Geeignete Polyester dieser Art sind beispielsweise Polyester aus Citronensäure und Ethylenglykol. Die beschriebenen Polyester haben in Waschflotten eine vergrauungsinhibierende Wirkung und verhindern die Wiederanlagerung von Schmutzteilchen aus der Waschflotte auf dem zu waschenden Textilgut.

Aus der prioritätsälteren, nicht vorveröffentlichten EP-A-0 433 010 sind u.a. Ester aus Citronensäure und Monosacchariden, Oligosacchariden und Methylglycosiden bekannt. Die Citronensäureester werden als Builder in Waschmittelformulierungen verwendet.

Der vorliegenden Erfindung liegt die Aufgabe zugrunde, neue Stoffe zur Verfügung zu stellen. Eine weitere Aufgabe der Erfindung besteht darin, gegenüber dem Stand der Technik verbesserte Zusätze für Wasch- und Reinigungsmittel zur Verfügung zu stellen.

Die Aufgabe wird erfindungsgemäß gelöst mit Estern aus Citronensäure oder Acetyl-Citronensäure-Anhydrid und Polyhydroxyverbindungen, die erhältlich sind durch Veresterung von
(a) Citronensäure und/oder Acetyl-Citronensäure-Anhydrid mit
(b) mindestens 3 OH-Gruppen enthaltenden Verbindungen aus der Gruppe Zuckercarbonsäuren, C₂- bis C₅-Alkylglucoside, Hydroxyalkylglucoside, Zuckeralkohole und Oxidationsprodukte von Oligosacchariden, die bis zu 4 Monosaccharideinheiten enthalten, Aminosorbit, Aminodisorbit, Glucosamin, N-Acetylglucosamin und Trishydroxyethylmelamin,
wobei jede OH-Gruppe der Verbindungen (b) im Mittel mit 0,15 bis 1 Molekül einer Verbindung (a) verestert ist. Die weitere Aufgabe wird gelöst mit der Verwendung von Citronensäure-Estern, die erhältlich sind durch Veresterung von
(a) Citronensäure und/oder Acetyl-Citronensäure-Anhydrid mit
(b) mindestens 3 OH-Gruppen enthaltenden Verbindungen aus der Gruppe Zuckercarbonsäuren, C₂- bis glucoside, Hydroxyalkylglucoside, Zuckeralkohole und Oxidationsprodukte von Oligosacchariden, die bis zu 4 Monosaccharideinheiten enthalten, Aminosorbit, Aminodisorbit, Glucosamin, N-Acetylglucosamin, Triethanolamin und Trishydroxyethylmelamin,
wobei jede OH-Gruppe der Verbindungen (b) im Mittel mit 0,15 bis 1 Molekül einer Verbindung (a) verestert ist, als Zusatz zu phosphatarmen oder phosphatfreien Wasch- und Reinigungsmitteln in Mengen von 0,1 bis 30 Gew.-%, bezogen auf die Formulierungen.

Die Herstellung der Citronensäure-Ester erfolgt durch Umsetzung der oben unter (a) und (b) beschriebenen Komponenten. Diese Reaktion wird üblicherweise unter Verwendung der bekannten sauren Katalysatoren für die Veresterung durchgeführt, z.B. Schwefelsäure, Paratoluolsulfonsäure, Benzolsulfonsäure oder Salzsäure. Sie kann aber auch prinzipiell ganz ohne Katalysator durchgeführt werden. Die Citronensäure kann auch, vorzugsweise bei der Umsetzung mit Naturstoffen oder deren Derivaten, vor der Veresterung teilweise neutralisiert werden, z.B. neutralisiert man 1 Mol Citronensäure mit 0,1 bis 2,9, vorzugsweise 0,5 bis 2,0 Mol einer einwertigen Base, z.B. NaOH. Die Veresterung kann in Substanz, d.h. durch Erhitzen der Komponenten (a) und (b) und Abdestillieren von Wasser oder auch in Gegenwart eines inerten Verdünnungsmittels vorgenommen werden. Das inerte Verdünnungsmittel, z.B. Toluol, Xylol oder isopropylbenzol, wirkt hierbei als sogenanntes Schleppmittel, um das bei der Veresterung gebildete Wasser azeotrop aus dem Reaktionsgemisch abzudestillieren. Die Veresterung kann bei Normaldruck oder auch unter vermindertem oder erhöhtem Druck vorgenommen werden. Bei der Veresterung in Gegenwart von inerten Lösemitteln kann gegebenenfalls in Gegenwart von Wasser-in-Öl-Emulgatoren gearbeitet werden. Diese Verbindungen erniedrigen die Viskosität des Reaktionsgemisches, so daß es gegen Ende der Veresterungsreaktion besser rührbar ist als emulgatorfreie Ansätze. Die Wasser-in-Öl-Emulgatoren werden in Mengen von 0 bis 5, vorzugsweise 0,05 bis 3 Gew.-%, bezogen auf den Gesamtansatz, verwendet. Geeignete Wasser-in-Öl-Emulgatoren sind beispielsweise Monoölsäureester von Triethanolamin, Sorbitanmonooleat und Sorbitandioleat.

Die Veresterung kann gegebenenfalls auch in Gegenwart von Polyalkylenglykolen mit mittleren Molekulargewichten (Zahlenmittel) von 200 bis 9000 durchgeführt werden. Insbesondere eignet sich hierfür Polyethylenglykol mit Molekulargewichten von 1500 bis 6000. Die Mengen an Polyalkylenglykolen betragen bis zu 60, vorzugsweise 10 bis 50 Gew.-%, bezogen auf den Gesamtansatz. Im Falle der Mitverwendung von Polyalkylenglykolen bei der Veresterung erfolgt auch eine zumindest partielle Veresterung dieser Verbindungen. Eine weitere Modifikation der Reaktionsprodukte ist dadurch möglich, daß die Veresterung in Gegenwart anderer Polycarbonsäuren, z.B. Äpfelsäure oder Aconitsäure, durchgeführt wird. Die Mengen betragen bis zu 20 Gew.-%, bezogen auf Citronensäure.

Die Veresterung der Reaktionspartner (a) und (b) kann kontinuierlich oder diskontinuierlich erfolgen. Man kann bei Raumtemperatur eine Mischung der Komponenten (a) und (b) herstellen, mit einem inerten Verdünnungsmittel versetzen und auf die Reaktionstemperatur erhitzen. In anderen Fällen kann es vorteilhaft sein, Zitronensäure vorzulegen und die Polyhydroxyverbindungen der Komponente (b) zuzudosieren. Bei der Veresterung erhält man überwiegend hochviskose Produkte, die in Form der freien Säure schwer wasserlöslich sind und je nach eingesetzter Alkoholkomponente farblos bis dunkel gefärbt sein können. Die Ester sind in Alkoholen, wie Methanol, Ethanol oder Butanol oder auch Tetrahydrofuran, löslich. Die Lösungen können beispielsweise mit Hilfe von Aktivkohle entfärbt werden. Der Verlauf der Veresterung kann mit Hilfe der Menge des abdestillierten wassers, der Säurezahl und der Verseifungszahl von Proben aus dem Reaktionsgemisch überwacht werden. Die Veresterung in Substanz erfolgt vorzugsweise in einem Knetreaktor. Allgemein führt man die Veresterung immer dann in einem Kneter durch, wenn relativ hochviskose Veresterungsprodukte entstehen. Je nach Verhältnis der Komponenten a) und b) und je nach Umsetzungsgrad bei der Veresterung lassen sich auch lineare oder verzweigte oligomere Ester aus mehreren Bausteinen gemäß a) und b) herstellen.

Als Zuckercarbonsäuren kommen die Oxidationsprodukte von Zuckern mit 4 bis 7 Kohlenstoffatomen in Betracht, Z.B. Gluconsäure, Glucoheptonsäure, Glucarsäure, Galactarsäure, Glucuronsäure oder Mannonsäure sowie die entsprechenden Lactone, z.B. Gluconolacton und Glucoheptonolacton.

Geeignete Verbindungen (b) sind außerdem Alkylglucoside und Alkylpolyglucoside, Alkylmaltoside und Alkylmaltotrioside. Die Alkylgruppe kann eine C₂- bis C₅-, vorzugsweise eine C₂- bis C₄-Alkylgruppe sein, z.B. Ethyl, n-Propyl, Isopropyl, n-Butyl oder Isobutyl. Außerdem kann die Alkylgruppe substituiert sein, z.B. eine Hydroxylgruppe tragen. Geeignete Verbindungen dieser Art sind beispielsweise Hydroxyethylglucosid und Hydroxypropylglucosid sowie die entsprechenden Polyglucoside. Die Polyglucoside enthalten im Mittel 1,1 bis 10, vorzugsweise 1,3 bis 3 Glucosideinheiten.

Oligosaccharide, die bis zu 4 Monosaccharideinheiten enthalten, sind beispielsweise Maltose, Maltotriose, Maltotetraose, Saccharose, Lactose, Leucrose, Isomaltulose, Chitobiose, Chitotriose, Chitotetraose und die davon durch Abspalten der Acetylgruppen erhältlichen Derivate. Als Monosaccharideinheiten der Oligosaccharide kommen alle üblichen Monosaccharide in Betracht, insbesondere von Glucose, Galactose, Fructose und Mannose abgeleitete Einheiten, Zuckeralkohole von Oligosacchariden, die bis zu 4 Monosaccharideinheiten enthalten, sind aus den obengenannten Oligosacchariden durch Reduktion erhältlich. Zu den Oxidationsprodukten der genannten Oligosaccharide gehört beispielsweise Saccharosetricarbonsäure und Lactobionsäure.

Weitere geeignete Verbindungen der Gruppe (b) sind Aminosorbit, Aminodisorbit, Glucosamin, N-Acetylglucosamin, Triethanolamin und Trishydroxyethylmelamin. Bevorzugt eingesetzte OH-Gruppen enthaltende Verbindungen der Komponente (b) sind Gluconsäure, Aminosorbit, Glucoheptonsäure. Maltose und Hydroxyethylglucosid.

Unmittelbar nach der Veresterung liegen die Citronensäure-Ester in der Säureform vor. Sie können durch Zusatz von Basen in die Salzform überführt werden. Geeignete Sasen sind beispielsweise Natriumnydroxid. Kaliumnydroxid, Lithiumnydroxid, Calciumhydroxid, Soda, Pottasche, Lithiumcarbonat, Calciumcarbonat, Ammoniumcarbonat, Ammoniumnydrogencarbonat, Ammoniak, Ethanolamin. Diethanolamin oder Triethanolamin. Die Basen werden vorzugsweise in Form der wäßrigen Lösungen eingesetzt. Von den genannten Basen verwendet man vorzugsweise Natronlauge, Natriumcarbonat oder Natriumhydrogencarbonat. Die Neutralisation der Citronensäure-Ester erfolgt bis zu einem solchen Ausmaß, daß die wäßrigen Lösungen der neutralisierten bzw. teilweise neutralisierten Citronensäure-Ester pH-Werte in dem Bereich von 3,0 bis 8,5, vorzugsweise 4,5 bis 7 haben. Die erfindungsgemäß zu verwendenden Citronensäure-Ester haben Säurezanlen von etwa 90 bis 780, vorzugsweise 265 bis 740 mg KOH/g Citronensäure-Ester. Die erfindungsgemäß zu verwendenden Citronensäureester haben ein Verhältnis von Säurezahl zu Verseifungszahl im Bereich von 0,33 bis 0,85, vorzugsweise 0,5 bis 0,8 bei Einsatz von säuregruppenfreien Polyhydroxyverbindungen gemäß b). Bei Einsatz säuregruppenhaltiger Polyhydroxyverbindungen gemäß b) muß bei der Herstellung der Polyester die so eingebrachte Säurezahl bei der Ermittlung des Verhältnisses von Säurezahl zu Verseifungszahl berücksichtigt werden, wobei sich die angegebenen Grenzen entsprechend verschieben. Die Natriumsalze der Ester können beispielsweise aus alkoholischer Lösung in Form amorpher Feststoffe isoliert werden, ebenso aus wäßriger Lösung durch Verdampfen des Wassers, vorzugsweise unter vermindertem Druck. Die Natriumsalze der Ester können auch durch Gefriertrockung oder Sprühtrocknung aus wäßriger Lösung oder durch Fällen mit Methanol oder Aceton gewonnen werden.

Um die Citronensäure-Ester ohne Veränderung zu lagern, werden sie nach der zumindest teilweise Vorgenommenen Neutralisation, bevorzugt in Form des neutralen Natriumsalzes, aus der wäßrigen Lösung gewonnen. Hierfür eignen sich die üblichen Verfahren, wie Gefriertrocknung, Sprühtrocknung oder Sprühwirbelschichttrocknung. Das Trocknen kann ohne weiteren Zusatz oder unter Abmischung mit waschaktiven Stoffen vorgenommen werden. Z.B. kann man durch Sprühwirbelschichttrocknung von Mischungen mit Aniontensiden, polymeren Waschmitteladditiven, Gerüststoffen oder Stellmitteln ein Granulat herstellen, das die erfindungsgemäß zu verwendenden Citronensäure-Ester enthält. Diese Art der Formulierung kann von entscheidendem Vorteil für den Einsatz der Citronensäure-Ester in alkalischen oder sauren Wasch-und Reinigungsmitteln sein.

Die oben beschriebenen Citronensäure-Ester werden als Zusatz zu pulverförmigen und flüssigen Waschmitteln verwendet, vorzugsweise in phosphatfreien oder phosphatarmen Waschmitteln, die nicht mehr als 25 Gew.-% Natriumtriphosphat enthalten. Die Einsatzmengen betragen 0,1 bis 30, vorzugsweise 0,5 bis 15 Gew.%, bezogen auf die Waschmittelformulierung. Die erfindungsgemäß zu verwendenden Citronensäure-Ester besitzen ein gutes Dispergiervermögen für Tonmineralien (Clay) in der Waschflotte. Diese Eigenschaft ist deshalb wichtig, weil lehmartige Verschmutzungen von Textilgut weit verbreitet sind. Die Citronensäure-Ester wirken in Waschmittelformulierungen als Builder, tragen zu einer Waschaktivierung der in den Wasch- und Reinigungsmittel enthaltenden Tenside bei und bewirken außerdem während des Waschvorgangs eine Reduktion der Inkrustierung auf dem gewaschenen Textilgut und leisten einen signifikanten Beitrag zu einer Schmutzdispergierung in der Waschflotte. Die Citronensäure-Ester sind zu einem hohen Maße biologisch abbaubar, z.B. betragen die Abbauraten mehr als 90 %.

Die Zusammensetzung von Waschmittelformulierungen kann sehr unterschiedlich sein. Gleiches gilt für die Zusammensetzung von Reinigungsmittelformulierungen. Wasch- und Reinigungsmittelformulierungen enthalten üblicherweise Tenside und gegebenenfalls Builder. Diese Angaben gelten sowohl für flüssige als auch für pulverförmige Wasch- und Reinigungsmittelformulierungen. Beispiele für die Zusammensetzung von Waschmittelformulierungen, die in Europa, in den USA und in Japan gebräuchlich sind, findet man beispielsweise in Chemical and Engn. News, Band 67, 35 (1989) tabellarisch dargestellt.

Die oben beschriebenen Citronensäure-Ester werden erfindungsgemäß in Waschmitteln eingesetzt, die bis zu 45 Gew.-% Phosphat enthalten, wobei die Verwendung der Citronensäure-Ester in Waschmitteln mit reduziertem Phosphatgehalt (darunter soll ein Phosphatgehalt von weniger als 25 Gew.% Natriumtriphosphat verstanden werden) oder in phosphatfreien Waschmitteln sowie in phosphatfreien Reinigungsmitteln bevorzugt ist. Die Citronensäure-Ester können dabei in Form eines Granulats, einer Paste, einer hochviskosen Masse, als Dispersion oder als Lösung in einem Lösemittel der Waschmittelformulierung zugegeben werden. Die Citronensäure-Ester können auch an der Oberfläche von Stellmitteln, z.B. Natriumsulfat oder Gerüststoffen (Zeolithen oder Bentoniten) sowie anderen festen Hilfsstoffen der Waschmittelformulierung adsorbiert werden.

Waschmittelformulierungen und Reinigungsmittelformulierungen sind pulverförmig oder flüssig. Sie können regional und gemäß dem speziellen Anwendungszweck verschieden zusammengesetzt sein.

Universalhaushaltswaschmittel für Trommelwaschmaschinen, wie sie in Europa weit verbreitet sind, enthalten gewöhnlich 5 bis 10 Gew.% Aniontenside; 1 bis 5 Gew.% nichtionische Tenside; 1 bis 5 Gew.% Schaumregulatoren, wie Silikonöle oder Seifen; 0 bis 40 Gew.% Enthärtungsmittel, wie Soda oder Pentanatriumtriphosphat, das durch die erfindungsgemäßen Verbindungen teilweise oder ganz ersetzt werden kann; 0 bis 30 Gew.% Ionenaustauscher, wie Zeolith A; 2 bis 7 Gew.% Natriumsilikate als Korrosionsinhibitoren; 10 bis 30 Gew.% Bleichmittel, wie Natriumperborat, Natriumpercarbonat organische Persäuren oder deren Salze; 0 bis 5 Gew.% Bleichaktivatoren, wie Tetraacetylethylendiamin, Pentaacetylglucose, Hexaacetylsorbit oder Acyloxibenzolsulfonat; Stabilisatoren, wie Magnesiumsilikat oder Ethylendiamintetraacetat; Vergrauungsinhibitoren, wie Carboximethylcellulose, Methyl- und Hydroxialkylcellulosen, mit Vinylacetat gepfropfte Polyglykole, oligomere und polymere Terephthalsäure/Ethylenglykol/Polyethylenglykol-Ester; Enzyme; optische Aufheller; Duftstoffe; Weichmacher; Farbstoffe und Stellmittel.

Im Gegensatz hierzu sind die Heavy Duty Detergents, die in den USA, Japan und diesen Ländern benachbarten Staaten in den Bottichwaschmaschinen verwendet werden, meist frei von Bleichmitteln, ihr Anteil an Aniontensiden ist dafür zwei bis dreimal so hoch, sie enthalten mehr Waschalkalien, wie Soda und Natriumsilikate (in der Regel bis zu 25 Gew.%) und zumeist fehlen ihnen auch die Bleichaktivatoren und Bleichstabilisatoren. Die Gehaltsangaben für Tenside und andere Inhaltsstoffe können sich noch beträchtlich erhöhen, wenn es sich um sogenannte Waschmittelkonzentrate handelt, die stellmittelfrei oder stellmittelarm in den Handel kommen. Fein- und Buntwaschmittel, Wollwaschmittel und Mittel für die manuelle Wäsche enthalten ebenfalls meist kein Bleichmittel und geringe alkalische Bestandteile bei entsprechend erhöhtem Tensidanteil.

Waschmittel für den gewerblichen Sektor sind auf die speziellen Verhältnisse des industriellen Waschens zugeschnitten (weiches Wasser, kontinuierliches Waschen), die es gestatten, schwerpunktmäßig auf die Art des Waschguts und der Verschmutzung einzugehen. Es werden daher Kombinationen verwendet, in denen ein Bestandteil vorherrscht oder andere ganz fehlen, die bei Bedarf getrennt zudosiert werden. Deshalb variieren die Bestandteile Tenside, Builder (Gerüststoffe), Alkalien und Bleichmittel dieser Waschmittel in weiten Grenzen.

Geeignete anionische Tenside für die vorgenannten Pulverwaschmittel sind beispielsweise Natriumalkylbenzolsulfonate, Fettalkoholsulfate und Fettalkoholpolyglykolethersulfate. Einzelne Verbindungen dieser Art sind beispielsweise C₈- bis C₁₂-Alkylbenzolsulfonate, C₁₂- bis C₁₆-Alkansulfonate, C₁₂- bis C₁₆-Alkylsulfate, C₁₂- bis C₁₆-Alkylsulfosuccinate und sulfatierte ethoxylierte C₁₂- bis C₁₆-Alkanole. Als anionische Tenside eignen sich außerdem sulfatierte Fettsäurealkanolamine, α-Sulfofettsäureester, Fettsäuremonoglyceride oder Umsetzungsprodukte von 1 bis 4 Mol Ethylenoxid mit primären oder sekundären Fettalkolen oder Alkylphenolen. Weitere geeignete anionische Tenside sind Fettsäureester bzw. Fettsäureamide von Hydroxy- oder Aminocarbonsäuren bzw. -sulfonsäuren, wie beispielsweise die Fettsäuresarkoside, -glykolate, -lactate, -tauride oder -isothionate. Die anionischen Tenside können in Form der Natrium-, Kalium- und Ammoniumsalze sowie als lösliche Salze organischer Basen, wie Mono-, Di- oder Triethanolamin oder andere substituierter Amine vorliegen. Zu den anionischen Tensiden gehören auch die üblichen Seifen, d.h. die Alkalisalze der natürlichen Fettsäuren.

Als nichtionische Tenside (Nonionics) sind z.B. Anlagerungsprodukte von 3 bis 40, vorzugsweise 4 bis 20 Mol Ethylenoxid an 1 Mol Fettalkohol, Alkylphenol, Fettsäure, Fettamin, Fettsäureamid oder Alkansulfonamid verwendbar. Die obengenannten Anlagerungsprodukte des Ethylenoxids können gegebenenfalls zusätzlich bis zu 90 Gew.-%, bezogen auf einkondensiertes Ethylenoxid und Propylenoxid, Propylenoxid einkondensiert enthalten. Die Anlagerungsprodukte, die Ethylenoxid und Propylenoxid einkondensiert enthalten, können gegebenenfalls durch Einkondensieren von Butylenoxid in Mengen bis zu 60 Gew.-%, bezogen auf den Gesamtgehalt an Alkylenoxid, modifiziert sein. Besonders wichtig sind die Anlagerungsprodukte von 5 bis 16 Mol Ethylenoxid an Kokos- oder Talgfettalkohole, an Oleylalkohol oder an synthetische Alkohole mit 8 bis 18, vorzugsweise 12 bis 18 C-Atomen, sowie an Mono- oder Dialkylphenole mit 6 bis 14 C-Atomen in den Alkylresten. Neben diesen wasserlöslichen Nonionics sind aber auch nicht bzw. nicht vollständig wasserlösliche Polyglykolether mit 1 bis 4 Ethylenglykoletherresten im Molekül von Interesse, insbesondere wenn sie zusammen mit wasserlöslichen nichtionischen oder anionischen Tensiden eingesetzt werden.

Weiterhin sind als nichtionische Tenside die wasserlöslichen, 20 bis 250 Ethylenglykolethergruppen und 10 bis 100 Propylenglykolethergruppen enthaltenden Anlagerungsprodukte von Ethylenoxid an Polypropylenglykolether, Alkylendiaminopolypropylenglykol und Alkylpolypropylenglykole mit 1 bis 10 C-Atomen in der Alkylkette brauchbar, in denen die Polypropylenglykoletherkette als hydrophober Rest fungiert.

Auch nichtionische Tenside vom Typ der Aminoxide oder Sulfoxide oder der Alkylpolyglucoside R-(Glu)ₓ, wobei R = C₈-C₁₆ [Glu=Glucose-Einheit] und x = 1-10, sind verwendbar.

Das Schaumvermögen der Tenside läßt sich durch Kombination geeigneter Tensidtypen steigern oder verringern. Eine Verringerung läßt sich ebenfalls durch Zusätze von nichttensidartigen organischen Substanzen erreichen.

Weitere Mischungsbestandteile von Waschmitteln können auch monomere, oligomere und polymere Phosphonate, Ethersulfonate auf der Basis von ungesättigten Fettalkoholen, z.B. Oleylalkoholethoxylatbutylether und deren Alkalisalze sein. Diese Stoffe können z.B. mit Hilfe der Formel RO(CH₂CH₂O)ₙ-C₄H₈-SO₃Na, in der n = 5 bis 40 und R = Oleyl ist, charakterisiert werden.

Die oben beschriebenen Citronensäure-Ester können auch als Zusatz zu Flüssigwaschmitteln verwendet werden. Bevorzugt in Flüssigwaschmitteln eingesetzte Citronensäure-Ester sind solche, die als Komponente (b) Polyglycerine mit 3 bis 10 Glycerin-Einheiten, Gluconsäure, Glucoheptonsäure, Maltose und Hydroxyethylglucosid einkondensiert enthalten. Die Flüssigwaschmittel enthalten als Abmischkomponente flüssige oder auch feste Tenside, die in der Waschmittelformulierung löslich oder zumindest dispergierbar sind. Als Tenside kommen hierfür die Produkte in Betracht, die auch in pulverförmigen Waschmitteln eingesetzt werden sowie flüssige Polyalkylenoxide bzw. polyalkoxylierte Verbindungen. Falls die Citronensäure-Ester mit den übrigen Bestandteilen des Flüssigwaschmittels nicht direkt mischbar sind, kann man mit Hilfe geringer Menge an Lösungsvermittlern, z.B. Wasser oder eines mit Wasser mischbaren organischen Lösemittel, z.B. Isopropanol, Methanol, Ethanol, Glykol, Diethylenglykol oder Triethylenglykol oder entsprechende Propylenglykole, homogene Mischungen herstellen. Die Tensidmenge in Flüssigwaschmitteln beträgt 4 bis 50 Gew.%, bezogen auf die gesamte Formulierung, da auch bei den Flüssigwaschmitteln je nach den regionalen Marktgegebenheiten oder dem Anwendungszweck die Anteile der Bestandteile in weiten Grenzen variieren.

Die Flüssigwaschmittel können Wasser in Mengen von 10 bis 60, vorzugsweise 20 bis 50 Gew.% enthalten. Sie können aber auch wasserfrei sein.

Wasserfreie Flüssigwaschmittel können auch Peroxoverbindungen zum Bleichen in suspendierter oder dispergierter Form enthalten. Als Peroxoverbindungen seien z.B. genannt: Natriumperborat, Peroxocarbonsäuren und Polymere mit teilweise peroxohaltigen Gruppen. Außerdem können die Flüssigwaschmittel gegebenenfalls Hydrotrope enthalten. Hierunter werden Verbindungen verstanden wie 1,2-Propandiol, Cumolsulfonat und Toluolsulfonat. Falls derartige Verbindungen zur Modifizierung der Flüssigwaschmittel eingesetzt werden, beträgt ihre Menge, bezogen auf das Gesamtgewicht des Flüssigwaschmittels, 2 bis 5 Gew.%. In vielen Fällen hat sich zur Modifizierung von pulverförmigen und flüssigen Waschmitteln auch ein Zusatz von Komplexbildnern als vorteilhaft erwiesen. Komplexbildner sind beispielsweise Ethylendiamintetraessigsäure, Nitrilotriacetat und Isoserindiessigsäure sowie Phosphonate, wie Aminotrismethylenphosphonsäure, Hydroxyethandiphosphonsäure, Ethylendiamintetraethylenphosphonsäure und deren Salze. Die Komplexbildner werden in Mengen von 0 bis 10 Gew.%, bezogen auf die Waschmittel, eingesetzt. Die Waschmittel können außerdem Zitrate, Di- oder Triethanolamin, Trübungsmittel, optische Aufheller, Enzyme, Parfümöle und Farbstoffe enthalten. Diese Stoffe sind, falls sie zur Modifzierung der Flüssigwaschmittel verwendet werden, zusammen in Mengen bis zu 5 Gew.% anwesend. Die Waschmittel sind vorzugsweise phosphatfrei. Sie können jedoch auch Phosphate enthalten, z.B. Pentanatriumtriphosphat und/oder Tetrakaliumpyrophosphat. Falls Phosphate eingesetzt werden, beträgt der Anteil der Phosphate an der Gesamtformulierung des Waschmittels bis zu 45, vorzugsweise bis zu 25 Gew.%.

Die erfindungsgemäß zu verwendenden Citronensäure-Ester können auch mit anderen bekannten Waschmitteladditiven (wie z.B. Vergrauungsinhibitoren, Claydispergatoren und Stoffen, die die Primärwaschwirkung verstärken, Farbübertragungsinhibitoren, Bleichaktivatoren) in Pulver- und Flüssigwaschmitteln (phosphathaltig und phosphatfrei) synergistische Effekte hervorrufen, bei denen nicht nur die Partikelschmutzdispergierung, sondern auch die Wirkung des anderen Waschmitteladditivs verstärkt werden kann.

Die Prozentangaben in den Beispielen bedeuten Gewichtsprozent.

### Beispiel 1

97,2 g einer 50,4 %igen wässrigen Lösung von Gluconsäure und 262,5 g Citronensäure-Monohydrat werden in einem 2 Liter fassenden Dreihalskolben mit 225 ml Xylol unter Rühren zum Sieden erhitzt. Mit Hilfe eines Wasserabscheiders destilliert man 93,6 g Wasser azeotrop ab. Danach dekantiert man das Xylol ab und entfernt das dem Reaktionsprodukt noch anhaftende Xylol destillativ unter vermindertem Druck. Das zurückbleibende viskose Produkt wird in 670 ml Methanol gelöst. Man erhielt eine 35 %ige methanolische Lösung eines Citronensäure-Esters mit einer Säurezahl von 210 mg KOH/g und einer Verseifungszahl von 285 mg KOH/g. Die methanolische Lösung wird mit Hilfe von Aktivkohle entfärbt und danach mit Sodalösung neutralisiert. Man erhielt das Natriumsalz eines Gluconsäure-Citronensäure-Esters mit einer Säurezahl von 10 mg KOH/g und einer Verseifungszahl von 209 mg KOH/g.

### Beispiel 2

128,3 g einer 50,4 %igen wässrigen Lösung von Gluconsäure und 210 g Citronensäure-Monohydrat werden unter Rühren auf eine Temperatur im Bereich von 120 bis 140°C erhitzt. Dabei destillieren 98,4 g Wasser ab. Um die Destillation zu beschleunigen, kann man sie unter vermindertem Druck, z.B. bei 400 mbar durchführen. Man erhält einen Ester mit einer Säurezahl von 535 mg KOH/g und einer Verseifungszahl von 756 mg KOH/g. Das Reaktionsprodukt wird anschließend in Wasser gelöst und durch Zugabe einer 20 %igen Sodalösung auf einen pH-Wert von 6,5 eingestellt. Nach der Gefriertrocknung der wässrigen Lösung erhält man das Natriumsalz des Esters mit einer Säurezahl von 13 mg KOH/g und einer Verseifungszahl von 188 mg KOH/g.

### Beispiel 3

90 g Maltose werden mit 525 g Citronensäure-Monohydrat und Toluol als Schleppmittel nach der in Beispiel 1 angegebenen Vorschrift umgesetzt. Nachdem etwa die Hälfte des bei der Reaktion entstehenden Wassers abdestilliert ist, gibt man zum Reaktionsgemisch 6 g des Monoölsäureesters von Triethanolamin (Wasser-in-Öl-Emulgator) zu. Dadurch ist das Reaktionsgemisch leichter rührbar. Das Umsetzungsprodukt hat eine Säurezahl von 539 mg KOH/g und eine Verseifungszahl von 789 mg KOH/g. Es wird in Wasser gelöst, mit Sodalösung auf pH 6,7 eingestellt und gefriergetrocknet. Die Verselfungszahl des Salzes beträgt 214 mg KOH/g.

### Beispiel 4

181 g Aminosorbit und 1.260 g Citronensäure-Monohydrat werden nach der in Beispiel 1 gegebenen Vorschrift umgesetzt. Man destilliert 216 g Wasser ab und erhält dabei ein Produkt mit einer Säurezahl von 512 mg KOH/g und einer Verseifungszahl von 745 mg KOH/g. Das daraus gewonnene Natriumsalz hat eine Säurezahl von <10 mg KOH/g und eine Verseifungszahl vor 201 mg KOH/g.

### Beispiel 5

34,5 g Aminodisorbit und 210 g Citronensäure-Monohydrat werden wie in Beispiel 1 beschrieben umgesetzt. Man destilliert 36 g Wasser ab und erhält dabei ein Produkt mit einer Säurezahl von 525 mg KOH/g und einer Verseifungszahl von 782 mg KOH/g. Das daraus hergestellte Natriumsalz hat eine Säurezahl von weniger als 10 mg KOH/g und eine Verseifungszahl von 234 mg KOH/g.

### Beispiel 6

90 g eines Hydroxyethylpolyglucosids mit einem Glucosidierungsgrad von 1,3 und 420 g Citronensäuremonohydrat werden wie in Beispiel 6 angegeben umgesetzt. Hydroxyethylpolyglucosid wurde nach der in Tenside Detergents Band 10, Heft 1, Seite 2 (1973) gegebenen Vorschrift hergestellt. Man destilliert 72 g Wasser ab. Das Umsetzungsprodukt hat eine Säurezahl von 518 mg KOH/g und eine Verseifungszahl von 760 mg KOH/g. Es wird in Wasser gelöst. mit Sodalösung auf pH 6,7 eingestellt und gefriergetrocknet. Die Verseifungszahl des Salzes beträgt 204 mg KOH/g.

### Anwendungstechnische Beispiele

### Clay-Dispergierung

Die Entfernung von Partikelschmutz von Gewebeoberflächen wird durch Zusatz von Polyelektrolyten unterstützt. Die Stabilisierung der nach der Ablösung der Partikel von der Gewebeoberfläche entstehenden Dispersion ist eine wichtige Aufgabe dieser Polyelektrolyte. Der stabilisierende Einfluß der anionischen Dispergiermittel ergibt sich dadurch, daß infolge von Adsorption von Dispergiermittelmolekülen auf der Feststoffoberfläche deren Oberflächenladung vergrößert und die Abstoßungsenergie erhöht wird. Weitere Einflußgrößen auf die Stabilität einer Dispersion sind ferner u.a. sterische Effekte, Temperatur, pH-Wert und die Elektrolytkonzentration.

Mit dem im folgenden beschriebenen Clay-Dispergiertest (CD-Test) kann auf einfache Weise die Dispergierfähigkeit verschiedener Polyelektrolyte beurteilt werden.

### CD-Test

Als Modell für partikulären Schmutz wird feingemahlener China-Clay SPS 151 benutzt. 1 g Clay wird unter Zusatz von 1 ml einer 0,1 %igen Natriumsalzlösung des Polyelektrolyten in 98 ml Wasser 10 Minuten in einem Standzylinder (100 ml) intensiv dispergiert. Sofort nach dem Rühren nimmt man aus der Mitte des Standzylinders eine Probe von 2,5 ml und bestimmt nach dem Verdünnen auf 25 ml die Trübung der Dispersion mit einem Turbidimeter. Nach 30- bzw. 60-minütiger Standzeit der Dispersion werden erneut Proben genommen und wie oben die Trübung bestimmt. Die Trübung der Dispersion wird in NTU (nephelometric turbidity units) angegeben. Je weniger sich die Dispersion während der Lagerung absetzt, um so höher sind die gemessenen Trübungswerte, und um so stabiler ist die Dispersion. Als zweite physikalische Meßgröße wird die Dispersionskonstante τ bestimmt, die das zeitliche Verhalten des Sedimentationsprozesses beschreibt. Da der Sedimentationsprozeß annähernd durch ein monoexponentielles Zeitgesetz beschrieben werden kann, gibt τ die Zeit an, in der die Trübung auf 1/e-tel des Ausgangszustandes zum Zeitpunkt t = 0 abfällt.

Je höher ein Wert für T ist, um so langsamer setzt sich die Dispersion ab.

Die gemäß den Beispielen hergestellten Natriumsalze der Ester wurden dem oben beschriebenen CD-Test unterworfen. Die dabei erhaltenen Ergebnisse sind in der folgenden Tabelle angegeben.

### Vergleichsbeispiel 1

Glycerintricitrat gemäß Beispiel 1 der DE-B-2 147 778.

### Vergleichsbeispiel 2

Sorbithexacitrat gemäß der DE-B-2 147 778

Für die in den Vergleichsbeispielen beschriebenen Verbindungen wurde ebenso ein CD-Test durchgeführt. Die dabei erhaltenen Ergebnisse sind in der Tabelle angegeben.

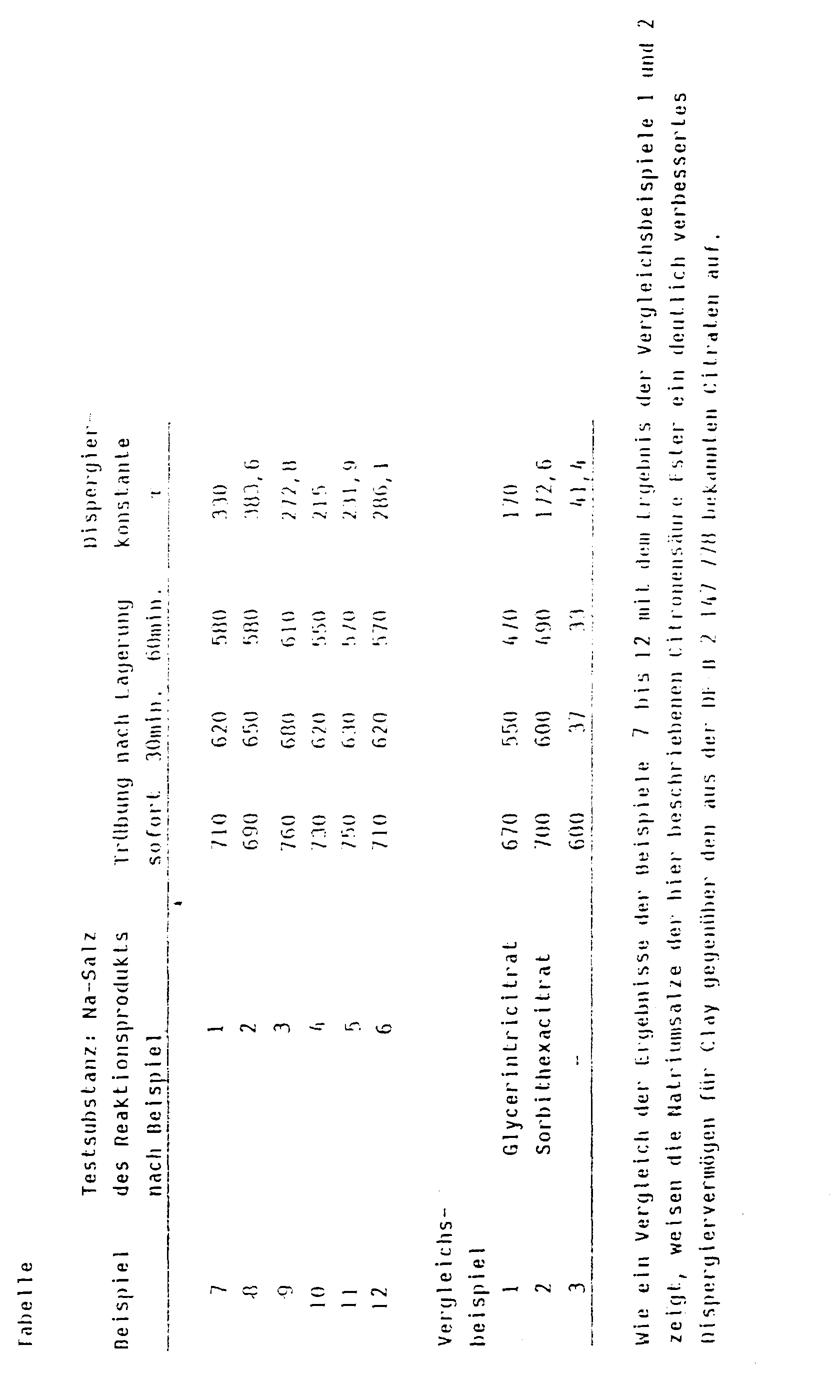

## Patentansprüche (Patentansprüche für folgende(n) Vertragsstaat(en): DE, FR, GB, IT)

1. Ester aus Citronensäure oder Acetylcitronensäureanhydrid und Polyhydroxyverbindungen, dadurch gekennzeichnet, daß sie erhältlich sind durch Veresterung von
(a) Citronensäure und/oder Acetylcitronensäureanhydrid mit
(b) mindestens 3 OH-Gruppen enthaltenden Verbindungen aus der Gruppe Zuckercarbonsäuren, C₂- bis C₅-AlKylglucoside, Hydroxyalkylglucoside, Zuckeralkohole und Oxidationsprodukte von Oligosacchariden, die bis zu 4 Monosaccharideinheiten enthalten, Aminosorbit, Aminodisorbit, Glucosamin, N-Acetylglucosamin und Trishydroxyethylmelamin,
wobei jede OH-Gruppe der Verbindungen (b) im Mittel mit 0,15 bis 1 Molekül einer Verbindung (a) verestert ist.

2. Verwendung von Citronensäure-Estern, die erhältlich sind durch Veresterung von
(a) Citronensäure und/oder Acetylcitronensäureanhydrid mit
(b) mindestens 3 OH-Gruppen enthaltenden Verbindungen aus der Gruppe Zuckercarbonsäuren, C₂- bis C₅-Alkylglucoside, Hydroxyalkylglucoside, Zuckeralkohole und Oxidationsprodukte von Oligosacchariden, die bis zu 4 Monosaccharideinheiten enthalten, Aminosorbit, Aminodisorbit, Glucosamin, N-Acetylglucosamin, Triethanolamin und Trishydroxyethylmelamin,
wobei jede OH-Gruppe der Verbindungen (b) im Mittel mit 0,15 bis 1 Molekül einer Verbindung (a) verestert ist, als Zusatz zu phosphatarmen oder phosphatfreien Wasch- und Reinigungsmitteln in Mengen von 0,1 bis 30 Gew.-%, bezogen auf die Formulierungen.

## Patentansprüche (Patentansprüche für folgende(n) Vertragsstaat(en): ES)

1. Verfahren zur Herstellung von Estern aus Citronensäure oder Acetylcitronensäureanhydrid und Polyhydroxyverbindungen, durch Veresterung von
(a) Citronensäure und/oder Acetylcitronensäureanhydrid mit
(b) mindestens 3 OH-Gruppen enthaltenden Verbindungen aus der Gruppe Zuckercarbonsäuren, C₂- bis C₅-Alkylglucoside, Hydroxyalkylglucoside, Zuckeralkohole und Oxidationsprodukte von Oligosacchariden, die bis zu 4 Monosaccarideinheiten enthalten, Aminosorbit, Aminodisorbit, Glucosamin, N-Acetylglucosamin und Trishydroxyethylmelamin,
wobei jede OH-Gruppe der Verbindungen (b) im Mittel mit 0,15 bis 1 Molekül einer Verbindung (a) verestert ist.

2. Verwendung von Citronensäure-Estern, die erhältlich sind durch Veresterung von
(a) Citronensäure und/oder Acetylcitronensäureanhydrid mit
(b) mindestens 3 OH-Gruppen enthaltenden Verbindungen aus der Gruppe Zuckercarbonsäuren, C₂- Gis C₅-Alkylglucoside, Hydroxyalkylglucoside, Zuckeralkohole und Oxidationsprodukte von Oligosacchariden, die bis zu 4 Monosaccharideinheiten enthalten, Aminosorbit, Aminodisorbit, Glucosamin, N-Acetylglucosamin, Triethanolamin und Trishydroxyethylmelamin,
wobei jede OH-Gruppe der Verbindungen (b) im Mittel mit 0,15 bis 1 Molekül einer Verbindung (a) verestert ist, als Zusatz zu phosphatarmen oder phosphatfreien Wasch- und Reinigungsmitteln in Mengen von 0,1 bis 30 Gew.-%, bezogen auf die Formulierungen.

## Claims (Claims for the following Contracting State(s): DE, FR, GB, IT)

1. Esters of citric acid or acetylcitric anhydride and polyhydroxy compounds, characterized in that they are obtainable by esterifying
(a) citric acid and/or acetylcitric anhydride with
(b) compounds of at least 3 OH groups selected from the group consisting of sugarcarboxylic acids, C₂-C₅-alkylglucosides, hydroxyalkylglucosides, sugar alcohols and oxidation products of oligosaccharides which contain up to 4 monosaccharide units, aminosorbitol, aminodisorbitol, glucosamine, N-acetylglucosamine, triethanolamine and trishydroxyethylmelamine,
each OH group of compounds (b) being on average esterified with from 0.15 to 1 molecule of a compound (a).

2. The use of citric esters obtainable by esterifying
(a) citric acid and/or acetylcitric anhydride with
(b) compounds of at least 3 OH groups selected from the group consisting of sugarcarboxylic acids, C₂-C₅-alkylglucosides, hydroxyalkylglucosides, sugar alcohols and oxidation products of oligosaccharides which contain up to 4 monosaccharide units, aminosorbitol, aminodisorbitol, glucosamine, N-acetylglucosamine, triethanolamine and trishydroxyethylmelamine,
each OH group of compounds (b) being on average esterified with from 0.15 to 1 molecule of a compound (a), as an additive in low-phosphate or phosphate-free washing and cleaning agent formulations in an amount of from 0.1 to 30% by weight, based on the formulations.

## Claims (Claims for the following Contracting State(s): ES)

1. A process for preparing esters of citric acid or acetylcitric anhydride and polyhydroxy compounds, characterized in that they are obtainable by esterifying
(a) citric acid and/or acetylcitric anhydride with
(b) compounds of at least 3 OH groups selected from the group consisting of sugarcarboxylic acids, C₂-C₅-alkylglucosides, hydroxyalkylglucosides, sugar alcohols and oxidation products of oligosaccharides which contain up to 4 monosaccharide units, aminosorbitol, aminodisorbitol, glucosamine, N-acetylglucosamine, triethanolamine and trishydroxyethylmelamine,
each OH group of compounds (b) being on average esterified with from 0.15 to 1 molecule of a compound (a).

2. The use of citric esters obtainable by esterifying
(a) citric acid and/or acetylcitric anhydride with
(b) compounds of at least 3 OH groups selected from the group consisting of sugarcarboxylic acids, C₂-C₅-alkylglucosides, hydroxyalkylglucosides, sugar alcohols and oxidation products of oligosaccharides which contain up to 4 monosaccharide units, aminosorbitol, aminodisorbitol, glucosamine, N-acetylglucosamine, triethanolamine and trishydroxyethylmelamine,
each OH group of compounds (b) being on average esterified with from 0.15 to 1 molecule of a compound (a), as an additive in low-phosphate or phosphate-free washing and cleaning agent formulations in an amount of from 0.1 to 30% by weight, based on the formulations.

## Revendications (Revendications pour l'(les) Etat(s) contractant(s) suivant(s): DE, FR, GB, IT)

1. Esters d'acide citrique ou d'anhydride acétylcitrique et de composés polyhydroxylés, caractérisés en ce qu'ils sont obtenus par estérification
a) d'acide citrique et/ou d'anhydride acétylcitrique avec
b) des composés contenant au moins 3 groupements OH, choisis dans le groupe des acides carboxyliques de sucre, des (alkyl en C₂-C₅)glucosides, des hydroxyalkylglucosides, des alcools de sucre et des produits d'oxydation d'oligosaccharides qui contiennent jusqu'à 4 motifs monosaccharide, de l'aminosorbitol, de l'aminodisorbitol, de la glucosamine, de la N-acétylglucosamine et de la trishydroxyéthylmélamine,
chaque groupement OH des composés b) étant estérifié en moyenne avec 0,15 à 1 molécule d'un composé a).

2. Utilisation d'esters d'acide citrique, qui sont obtenus par estérification
a) d'acide citrique et/ou d'anhydride acétylcitrique avec
b) des composés contenant au moins 3 groupements OH, choisis dans le groupe des acides carboxyliques de sucre, des (alkyl en C₂-C₅)glucosides, des hydroxyalkylglucosides, des alcools de sucre et des produits d'oxydation d'oligosaccharides qui contiennent jusqu'à 4 motifs monosaccharide, de l'aminosorbitol, de l'aminodisorbitol, de la glucosamine, de la N-acétylglucosamine, de la triéthanolamine et de la trishydroxyéthylmélamine,
chaque groupement OH des composés b) étant estérifié en moyenne avec 0,15 à 1 molécule d'un composé a), comme additif à des produits de lavage et de nettoyage pauvres en phosphates ou exempts de phosphates, dans des proportions de 0,1 à 30% en poids par rapport aux compositions.

## Revendications (Revendications pour l'(les) Etat(s) contractant(s) suivant(s): ES)

1. Procédé de préparation d'esters d'acide citrique ou d'anhydride acétylcitrique et de composés polyhydroxylés, caractérisé en ce qu'ils sont obtenus par estérification
a) d'acide citrique et/ou d'anhydride acétylcitrique avec
b) des composés contenant au moins 3 groupements OH, choisis dans le groupe des acides carboxyliques de sucre, des (alkyl en C₂-C₅)glucosides, des hydroxyalkylglucosides, des alcools de sucre et des produits d'oxydation d'oligosaccharides qui contiennent jusqu'à 4 motifs monosaccharide, de l'aminosorbitol, de l'aminodisorbitol, de la glucosamine, de la N-acétylglucosamine et de la trishydroxyéthylmélamine,
chaque groupement OH des composés b) étant estérifié en moyenne avec 0,15 à 1 molécule d'un composé a).

2. Utilisation d'esters d'acide citrique, qui sont obtenus par estérification
a) d'acide citrique et/ou d'anhydride acétylcitrique avec
b) des composés contenant au moins 3 groupements OH, choisis dans le groupe des acides carboxyliques de sucre, des (alkyl en C₂-C₅)glucosides, des hydroxyalkylglucosides, des alcools de sucre et des produits l'oxydation d'oligosaccharides qui contiennent jusqu'à 4 motifs monosaccharide, de l'aminosorbitol, de l'aminodisorbitol, de la glucosamine, de la N-acétylglucosamine, de la triéthanolamine et de la trishydroxyéthylmélamine,
chaque groupement OH des composés b) étant estérifié en moyenne avec 0,15 à 1 molécule d'un composé a),
comme additif à des produits de lavage et de nettoyage pauvres en phosphates ou exempts de phosphates, dans des proportions de 0,1 à 30% en poids par rapport aux compositions.
